# DEMANDE DE BREVET EUROPEEN

(11) **EP 0 748 654 A1**
(43) Date de publication de la demande: **18.12.1996**
(21) Numéro de dépôt: 96401248.8
(22) Date de dépôt: 10.06.1996
(51) Int. Cl.: B01J 31/02, C07C 2/60

(54) **Catalyseur d'alkylation aliphatique comprenant une phase active comportant un composé cuivreux, sur support**

(30) Priorité: 15.06.1995 FR 9507270; 15.06.1995 FR 9507271
(71) Demandeur: INSTITUT FRANCAIS DU PETROLE, 92502 Rueil-Malmaison (FR)
(72) Inventeur: Benazzi, Eric, 78360 Montesson (FR); Ferrer, Nathalie, 78400 Chatou (FR); Chauvin, Yves, 78230 Le Pecq (FR); Olivier, Hélène, 92500 Rueil Malmaison (FR); Hirschauer, André, 78360 Montesson (FR); Bernhard, Jean-Yves, 91540 Mennecy (FR)

(57) **Abrégé**

L'invention concerne un catalyseur comprenant un support poreux organique ou minéral et au moins une phase active comprenant au moins un halogénure d'aluminium, au moins un composé choisi parmi les halogénures d'ammonium quaternaire et les halohydrates d'amine, et au moins un composé cuivreux, ledit support ayant été imprégné par ladite phase active, ledit catalyseur étant tel qu'il est constitué essentiellement de particules de diamètre moyen compris entre 0,1 et 150 µm, et en ce que ledit support, avant son imprégnation par ladite phase active, possède un volume poreux total compris entre 0,1 et 6 cm³/g.

## Description

La présente invention concerne un catalyseur comprenant un support poreux organique ou minéral et au moins une phase active constituée d'au moins un halogénure d'aluminium, d'au moins un halohydrate d'amine et/ou au moins d'un halogénure d'ammonium quaternaire et d'au moins un composé cuivreux Cu(l). L'invention concerne aussi l'utilisation en alkylation dite aliphatique dudit catalyseur c'est-à-dire en alkylation catalytique d'isobutane et/ou d'isopentane au moyen d'au moins une oléfine comprenant de 2 à 6 atomes de carbone par molécule, (c'est-à-dire d'une oléfine C₂-C₆), ce qui permet d'obtenir des composés paraffiniques à haut degré de ramification et à haut indice d'octane.

Un grand nombre de catalyseurs acides, liquides ou solides, sont connus pour réaliser l'alkylation aliphatique d'isoparaffine(s) telles que l'isobutane ou l'isopentane, par au moins une oléfine telle que le propylène, les butènes-1 et -2, l'isobutène. Les catalyseurs les plus couramment utilisés dans la pratique industrielle sont l'acide sulfurique concentré et l'acide fluorhydrique seul ou en mélange avec des acides de Lewis tels que le trifluorure de bore. Ces procédés souffrent d'inconvénients majeurs : l'acide fluorhydrique en raison de sa toxicité et de sa forte volatilité; l'acide sulfurique en raison d'une consommation importante du catalyseur nécessitant un retraitement onéreux. C'est pourquoi on a préconisé l'emploi de catalyseurs solides ou supportés sur des solides tels que les aluminosilicates ou les oxydes métalliques tels que la zircone traitée par l'acide sulfurique.

Il a été proposé dans la demande de brevet européen EP-A-0.553.009 d'utiliser pour catalyser la réaction d'alkylation aliphatique les complexes ioniques liquides que font les halogénures d'aluminium avec certains halogénures d'ammonium quaternaires ou avec certains halohydrates d'amines, sur support poreux organique ou minéral. Ces complexes, dits encore "sels fondus", ont été décrits par C.H. Hussey dans *Advances in Molten Salts Chemistry,* vol. 5, p185, Elsevier, New-York, 1985 et par C.A. Angell et J.W. Shuppert dans J. Phys. Chem. 84, 538, 1980 et font l'objet des brevets français FR-A-2.626.572 et FR-A-2.692.888.

La présente invention concerne un catalyseur comprenant un support poreux organique ou minéral, de préférence de la silice, et une phase active comprenant au moins un halogénure d'aluminium, au moins un halogénure d'ammonium quaternaire et/ou au moins un halohydrate d'amine, c'est-à-dire au moins un composé choisi parmi les halohydrates d'amine et les halogénures d'ammonium quatemaires, et au moins un composé cuivreux, ledit support ayant été imprégné par ladite phase active, ledit catalyseur étant tel qu'il est constitué essentiellement de particules de diamètre moyen compris entre 0,1 et 150 µm et de préférence entre 5 et 110 µm et de manière encore plus préférée entre 5 et 80 µm, et en ce que ledit support, avant son imprégnation par ladite phase active, possède un volume poreux total compris entre 0,1 et 6 cm³/g, de préférence entre 0,2 et 5,5 cm³/g, de façon encore plus préférée entre 0,3 et 5 cm³/g.

Le catalyseur selon la présente invention conduit de façon surprenante à des performances catalytiques améliorées par rapport à celles décrites dans la demande de brevet européen EP-A-0.553.009. En effet il a été découvert que de façon inattendue l'addition de composés de cuivre (I) et notamment d'halogénures cuivreux aux complexes ioniques décrits dans ladite demande de brevet améliorait la sélectivité et la stabilité du catalyseur, permettait d'éviter l'introduction de protons additionnels pour réactiver le catalyseur et diminuait la teneur en chlore des alkylats, produits de la réaction.

D'autre part, la demande de brevet européen EP-A-0.709.356 décrit une composition catalytique résultant du mélange d'au moins un halogénure d'aluminium, d'au moins un halogénure d'ammonium quaternaire et/ou au moins un halohydrate d'amine et d'au moins un composé cuivreux, de préférence halogénure. Ladite demande décrit également un procédé pour l'alkylation des isoparaffines par les oléfines avec cette composition. La présente demande décrit donc un catalyseur comprenant la composition catalytique de la demande de brevet européen EP-A-0.709.356 sur support.

Le catalyseur de la présente invention renferme un support poreux organique ou minéral, de préférence la silice, et au moins une phase active comprenant au moins un halogénure d'aluminium, au moins un halohydrate d'amine et/ou au moins un halogénure d'ammonium quaternaire, de préférence un chlorhydrate ou un bromhydrate d'amine, et au moins un composé cuivreux Cu(l). Le support est imprégné par au moins une phase active dont la composition a été décrite précédemment. Ladite phase active est un complexe ionique non miscible à la phase hydrocarbure communément appelé "sel fondu". Elle peut être obtenue liquide à température ambiante, mais aussi à température plus élevée de préférence inférieure à 150°C et de manière encore plus préférée inférieure à 100°C. Dans tous les cas, elle est liquide lors de son imprégnation sur le support.

L' halogénure d'aluminium plus particulièrement utilisable dans la présente invention est choisi dans le groupe formé par les trifluorures, les trichlorures et les tribromures d'aluminium tels que AlCl₃, AlBr₃. Il peut être mis en oeuvre pur ou en mélange, par exemple, dans des proportions variables allant de 99% de l'un des halogénures jusqu'à 99% de l'autre dans le cas du mélange de deux halogénures d'aluminium. On peut ainsi par exemple mélanger AlCl₃ avec AlBr₃.

L'halogénure d'ammonium quaternaire utilisable dans la présente invention est choisi dans le groupe formé par les sels d'ammonium quaternaires, acycliques ou faisant partie d'un cycle, qui répondent généralement aux formules générales suivantes:

R¹R²R³R⁴N⁺ X⁻ (I)

R¹R²N⁺=CR³R⁴⁺ X⁻ (II)

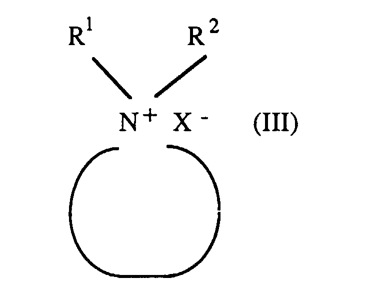

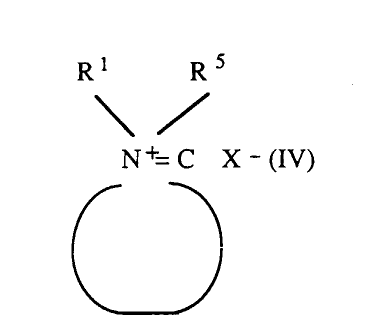

dans lesquelles R¹, R², R³, R⁴ ou R⁵, identiques ou différents, représentent chacun des restes hydrocarbyles comprenant généralement 1 à 12 atomes de carbone, par exemple alkyl, cycloalkyl, aryl, aralkyl, R⁵ pouvant être également l'hydrogène ou des restes hydrocarbyles substitués comprenant par exemple d'autres atomes comme l'azote.

Des radicaux tels que R⁶ peuvent unir deux molécules telles que ci-dessus comme, par exemple R¹R²N+=CR³-R⁶-CR³=N+R¹R² (X⁻)₂, R⁶ pouvant être un reste alkylène ou encore phénylène.

Les cycles III et IV sont constitués de 4 à 10 atomes, de préférence de 5 à 6 atomes qui peuvent comprendre, outre l'azote de l'ammonium quaternaire, des atomes de carbone ou éventuellement d'autres atomes d'azote, 1 ou 2 généralement.

Parmi les groupements R¹, R², R³, R⁴ ou R⁵ on mentionnera les radicaux méthyl, éthyl, propyl, isopropyl, butyl, secondaire butyl, tertiaire butyl, amyl, méthylène, éthylidène, phényl ou benzyl; R⁶ pourra être un groupement méthylène, éthylène, propylène ou phénylène.

Dans ces formules X représente un ion halogénure, par exemple l'ion bromure ou l'ion chlorure.

A titre d'exemples de sels d'ammonium utilisables selon l'invention on peut citer plus particulièrement les sels d'imidazolium et de pyridinium tels que le chlorure de N-butylpyridinium, le bromure d'éthylpyridinium, le chlorure de butyl-1 méthyl-3 imidazolium, le chlorure de diéthylpyrazolium, le chlorure d'éthyl-1 méthyl-3 imidazolium.

L'halohydrate d'amine, de préférence le chlorhydrate ou le bromhydrate d'amine, utilisable dans le cadre de l'invention, comporte de préférence une mole d'acide halohydrique, de préférence chlorhydrique ou bromhydrique, par mole d'amine mais peuvent également comporter deux moles d'acide par mole d'amine. On peut aussi utiliser des mélanges d'halohydrates à une mole et à deux moles d'acide halohydrique. L'halohydrate dérive d'amine ou de diamine acyclique ou d'amine faisant partie d'un cycle qui peut comporter un ou plusieurs atomes d'azote et qui répond aux formules générales suivantes:

NR'¹R'²R'³ (I)

R'¹N=CR'²R'³ (II)

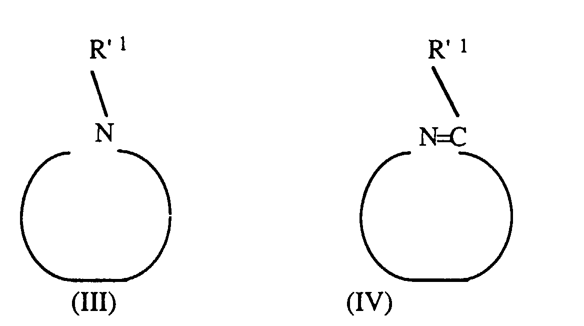

dans lesquelles R'¹,R'²,R'³, identiques ou différents, représentent des restes hydrocarbyles comportant généralement 1 à 12 atomes de carbone par exemple alkyl, cycloalkyl, aryl, aralkyl. L'un de ces substituants R'¹, R'², R'³ peut être l'hydrogène. Les cycles III et IV sont généralement constitués de 4 à 10 atomes, de préférence de 5 à 6 atomes qui peuvent comprendre, outre un ou des atomes d'azote, des atomes de carbone liés par des liaisons simples ou doubles. Les cycles III et IV peuvent être condensés avec d'autres cycles et porter des substituants tels que definis ci-dessus, des fonctions amines, des atomes de fluor, de chlore, de brome.

Parmi les groupements R'¹, R'² et R'³ on mentionnera les radicaux méthyl, éthyl, propyl, isopropyl, butyl, sec-butyl, amyl, méthylène, éthylidène, phényl, benzyl. Les cycles tels que IV sont représentés par les familles des pyridines, des imidazoles, des triazines, des pyrazoles, des pyrimidines, des triazoles.

De manière préférée, l'halohydrate est choisi dans le groupe formé par les chlorhydrates ou les bromhydrates de la pyridine, des picolines-2, -3 ou -4, des lutidines, de l'éthyl-2pyridine, de l'isopropyl-3 pyridine, des chloro-2 ou-4 pyridine, de la N,N-diméthylamino-4 pyridine, du N-méthylimidazole, du N-butylimidazole, de la pipéridine, de la N-méthylimidazoline.

Un des moyens essentiels déterminants selon l'invention est la présence du cuivre (I) dans la phase active sur support.

Le composé du cuivre (I) utilisable dans le cadre de l'invention est généralement choisi dans le groupe formé par les halogénures cuivreux, l'acétate cuivreux, le sulfate cuivreux, le nitrate cuivreux et le perchlorate cuivreux. De préférence, ledit composé est un halogénure cuivreux, ce qui évite l'introduction d'ions supplémentaires dans le milieu réactionnel servant à la réalisation de la phase active. L'halogénure de cuivre encore plus préféré dans le cadre de l'invention est choisi dans le groupe formé par le chlorure cuivreux et le bromure cuivreux.

Les composants de la phase active, tels que définis ci-dessus sont, de préférence, mis en oeuvre dans des rapports molaires [halogénure d'aluminium:(halogénure d'ammonium quaternaire et/ou halohydrate d'amine)] compris entre 1,1:1 et 3,5:1, de préférence entre 2:1 et 3:1, et (halogénure d'aluminium:halogénure cuivreux) compris entre 1:0,1 et 1:1, de préférence entre 1:0,2 et 1:0,5.

Les composés entrant dans la composition de la phase active selon l'invention, qui est ensuite imprégnée sur le support, peuvent être mélangés dans un ordre quelconque, à une température comprise entre -20°C et + 80°C. Le mélange peut se faire par une simple mise en contact suivie d'une agitation jusqu'à formation d'un liquide ou d'une suspension manipulable. On peut avantageusement, dans le cas où le composé cuivreux est un halogénure cuivreux, réaliser l'opération en ne mélangeant qu'environ un tiers de l'halogénure d'aluminium à l'halogénure d'ammonium quaternaire et/ou l'halohydrate d'amine, le composé obtenu solubilisant facilement le composé cuivreux, puis en ajoutant par portions les deux autres tiers restants d'halogénure d'aluminium. Cette façon d'opérer évite l'emploi de solvants basiques facilitant la dissolution du composé cuivreux. De cette façon on obtient une préparation homogène facilement manipulable et imprégnable sur le support.

Lorsque la silice est utilisée comme support, elle peut contenir des impuretés comme par exemple les oxydes, les alcalins, les alcalino-terreux, les composés d'aluminium ou toute autre impureté connue de l'homme du métier, la quantité totale de ces impuretés n'excédant généralement pas 2 % en poids par rapport à la silice

Le support poreux organique ou minéral, de préférence la silice, est généralement tel que, avant son imprégnation par la phase acide, sa surface spécifique est comprise entre 0,1 et 1500 m²/g, et son volume poreux total est compris entre 0,1 et 6 cm³/g, de préférence entre 0,2 et 5,5 cm³/g et de façon encore plus préférée entre 0,3 et 5 cm³/g. De plus, il est généralement constitué essentiellement de particules de diamètre moyen compris entre 0,1 et 150 µm, de préférence entre 5 et 110 µm, et de manière encore plus préférée entre 5 et 80 µm.

La phase active (encore appelée "sel fondu") occupe généralement entre 80 et 100 % du volume poreux total du support, et de préférence entre 90 et 100 % dudit volume poreux.

La teneur pondérale du catalyseur en phase active est comprise entre 1 et 48% et de préférence entre 10 et 48% poids.

Le procédé de préparation du catalyseur selon l'invention comprend généralement au moins deux étapes. Dans une première étape le support poreux organique ou minéral est calciné à une température supérieure à 50 °C, de préférence supérieure à 80 °C et de manière encore plus préférée comprise entre 150 et 600°C, par exemple égale à environ 500° C. La durée de cette étape de calcination est habituellement comprise entre 10 minutes et 50 heures, de préférence entre 15 minutes et 25 heures. La calcination est généralement effectuée en présence d'air sec ou de mélange air sec/azote, de débit compris entre 0,001 et 10 I/h/g, de préférence entre 0,1 et 5 l/h/g. La deuxième étape consiste en l'imprégnation dudit support calciné, par la phase active. Pour réaliser cette deuxième étape, on peut utiliser toutes les techniques connues de l'homme du métier. A ce procédé de préparation s'ajoute généralement une étape supplémentaire de préparation de la phase active, préalable à l'étape d'imprégnation.

Le catalyseur selon la présente invention est mis en oeuvre dans un procédé qui permet de réaliser dans les meilleures conditions la réaction d'alkylation de l'isoparaffine par les oléfines. En particulier, ladite réaction étant caractérisée par une forte exothermicité (environ 83,6 kJ/mol de butène transformé si l'oléfine est le butène et si l'isoparaffine est l'isobutane), la mise en oeuvre du catalyseur selon la présente invention permet d'obtenir une bonne homogénéité de température et de concentration en réactifs.

Dans le procédé d'alkylation de l'isoparaffine utilisant le catalyseur selon la présente invention, les conditions opératoires, et plus particulièrement la température et la pression, sont généralement choisies de façon à ce que le mélange constitué par l'isopraffine, l'(les) oléfine(s) et les produits de la réaction soit liquide. De plus, il est important que le catalyseur soit immergé dans ledit liquide de façon à assurer un bon contact liquide-solide.

Le catalyseur selon l'invention est avantageusement mis en oeuvre dans la zone réactionnelle d'alkylation de l'isobutane et/ou de l'isopentane avec au moins une oléfine comprenant de 2 à 6 atomes de carbone par molécule, en phase liquide et en mélange avec l'isoparaffine et/ou un mélange d'isoparaffines. Le catalyseur selon l'invention peut être mis en oeuvre en lit expansé, en zone réactionnelle presque parfaitement agitée ou en lit circulant, et de préférence il est mis en oeuvre dans un procédé qui utilise une phase liquide continue, le catalyseur pouvant être utilisé sous forme de suspension selon les deux mises en oeuvre préférées décrites ci-après.

Une première mise en oeuvre préférée du catalyseur selon la présente invention est la zone réactionnelle à mélange presque parfait, c'est-à-dire à mélange parfait ou s'en rapprochant (cuve agitée ou Grignard), utilisant au moins un moyen d'agitation par exemple au moins une hélice, de façon à obtenir une agitation suffisante du catalyseur en suspension dans la phase liquide hydrocarbonée, laquelle comprend généralement l'isoparaffine (isobutane et/ou isopentane), au moins une oléfine, éventuellement au moins un diluant inerte (par exemple le propane et le normal butane) et les produits de la réaction d'alkylation. La charge à convertir, constituée d'isobutane et/ou d'isopentane et d'au moins une oléfine, peut être par exemple introduite sous forme liquide en au moins un point au sein de la phase liquide hydrocarbonée présente dans la zone réactionnelle.

Une deuxième mise en oeuvre préférée du catalyseur selon la présente invention en suspension dans une phase hydrocarbonée est le lit mobile à co-courant c'est-à-dire le lit circulant. Dans cette mise en oeuvre le catalyseur en suspension dans la phase liquide hydrocarbonée, comprenant généralement l'isoparaffine (isobutane et/ou isopentane), au moins une oléfine, éventuellement au moins un diluant inerte (par exemple le propane ou le normal butane) et les produits de la réaction d'alkylation, circule de bas en haut dans la zone réactionnelle. L'ensemble constitué par la suspension de catalyseur dans la phase hydrocarbonée circule ensuite au travers d'au moins un échangeur de chaleur et d'au moins une pompe, avant d'être de nouveau introduit à l'entrée de la zone réactionnelle. La charge à convertir, constituée d'isobutane et/ou d'isopentane et d'au moins une oléfine, est introduite soit sous forme liquide, soit sous forme gazeuse en au moins un point de la zone réactionnelle.

Dans les deux types de mises en oeuvre décrites précédemment, l'isoparaffine (isobutane et/ou isopentane) n'ayant pas été convertie ou ayant été introduite en excès par rapport à la stoechiométrie de la réaction, est généralement recyclée après séparation de l'alkylat, soit par introduction directe dans la zone réactionnelle, soit par mélange avec la charge à convertir.

Le mélange isoparaffine(s)-oléfine(s) est généralement introduit dans la zone réactionnelle à une vitesse spatiale horaire, exprimée en poids d'oléfine introduite par unité de poids du catalyseur et par heure (pph) comprise entre 0,001 et 10 h⁻¹ et de préférence comprise entre 0,002 et 2 h⁻¹. Ledit mélange peut aussi être réalisé à l'intérieur de la zone réactionnelle. Dans tous les cas le mélange ainsi constitué est dans la zone réactionnelle dans des conditions de pression et de température telles que le mélange d'hydrocarbures reste liquide sur le catalyseur.

La température de réaction est généralement comprise entre -40°C et + 80°C, de préférence entre -20°C et +25°C. La pression de la zone réactionnelle est généralement suffisante pour maintenir les hydrocarbures à l'état liquide dans ladite zone.

Afin de limiter les réactions secondaires, on utilise généralement un excès d'isoparaffine par rapport à l'oléfine. A titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butène(s) dans la charge est généralement compris entre 1 et 100, de préférence entre 3 et 50 et de manière souvent préférée entre 5 et 15.

Lorsque la nature du catalyseur et les conditions de réaction sont judicieusement choisies (en particulier la température), le catalyseur selon l'invention permet la production de produits d'alkylation d'au moins une isoparaffine par au moins une oléfine qui sont intéressants comme carburants pour les moteurs et constituants d'essence et qui comprennent par exemple au moins 60 % moles de paraffines possédant 8 atomes de carbone par molécule et moins de 1 % moles de composés non saturés, les paraffines comprenant 8 atomes de carbone par molécule comprenant 70 à 98 % en moles de triméthylpentanes.

Un autre avantage du catalyseur selon la présente invention est la possibilité d'alkyler, à basse température, l'isobutane avec des mélanges d'oléfines comportant de 2 à 6 atomes de carbone par molécule, où la proportion d'oléfines comportant plus de 4 atomes de carbone par molécule est très importante.

Les exemples suivants illustrent l'invention sans toutefois en limiter la portée.

### EXEMPLE N° 1

### Préparation du catalyseur 1 selon l'invention

Dans un ballon en verre muni d'un barreau magnétique, purgé d'air et d'humidité et maintenu à 10°C on introduit successivement 13,7 g de chlorure de butyl-1 méthyl-3 imidazolium, 20 ml d'heptane et par portions 8,5 g de chlorure d'aluminium fraîchement sublimé. On obtient ainsi une composition liquide qu'on introduit dans un autre ballon contenant 4,7 g de chlorure cuivreux anhydre qui se dissout lentement dans le mélange ionique. A cette solution on introduit par portions 14,5 g de chlorure d'aluminium qui se dissout lentement dans la composition liquide.

On active 16 g de silice de surface spécifique égale à 395 m2/g, de volume poreux total égal à 2,5 cm3/g et de diamètre moyen des particules égal à 35 µm, par calcination sous air sec pendant 4 heures à 500°C. La silice ainsi activée est conservée sous argon. On procède alors à une imprégnation à sec et à l'abri de l'humidité, de 14 g de ladite silice par 43,9 g de phase active préparée selon le mode opératoire décrit ci-dessus.

Le solide ainsi obtenu, appelé catalyseur 1, possède une teneur pondérale en phase active égale à 75,8 % poids.

### Alkylation de l'isobutane par le butène-2 avec le catalyseur 1

Le catalyseur 1 est utilisé pour alkyler l'isobutane par le butène-2 de façon à produire des paraffines ramifiées à hauts indices d'octane. On introduit 18 g du catalyseur 1, préparé ci-dessus, dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20 °C.

100 cm³ d'isobutane sont alors ajoutés dans le réacteur contenant le catalyseur sous agitation, ledit réacteur étant immergé dans un bain froid à -6 °C. On laisse sous agitation le système (catalyseur + isobutane) pendant une durée de 30 minutes afin d'homogénéiser la température.

On ajoute en continu un mélange d'isobutane et de butène-2, contenant 25 % poids de butène-2, pendant une durée totale de 8 heures, la température du réacteur étant maintenue à -5 °C pendant toute la durée de l'injection. Le débit volumique du mélange (isobutane + butène-2) est égal à 25 ml/h.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 1. La conversion de l'oléfine est de 100 %.

### Exemple n°2

### Préparation du catalyseur 2 comparatif

Dans un ballon en verre muni d'un agitateur magnétique, purgé d'air et d'humidité et maintenu à 10°C on a introduit successivement 23 g de chlorure d'aluminium fraîchement sublimé, 20 ml d'heptane et par portions 13,7 g de chlorure de butyl-1 méthyl-3 imidazolium. On obtient ainsi une composition liquide.

On active 16 g de silice de surface spécifique égale à 395 m2/g et de volume poreux total égal à 2,5 cm3/g et de diamètre moyen des particules égal à 35 µm, par calcination sous air sec pendant 4 heures à 500°C. La silice ainsi activée est conservée sous argon. On procède alors à une imprégnation à sec et à l'abri de l'humidité, de 14 g de ladite silice par 43,9 g de phase active préparée selon le mode opératoire décrit ci-dessus.

Le solide ainsi obtenu, appelé catalyseur 2, possède une teneur pondérale en phase active égale à 75, 8% poids.

Le catalyseur 2 ainsi préparé est testé dans les mêmes conditions opératoires que celles décrites dans l'exemple 1. Les résultats catalytiques obtenus sont donnés dans le tableau 1. La conversion de l'oléfine est de 100%.

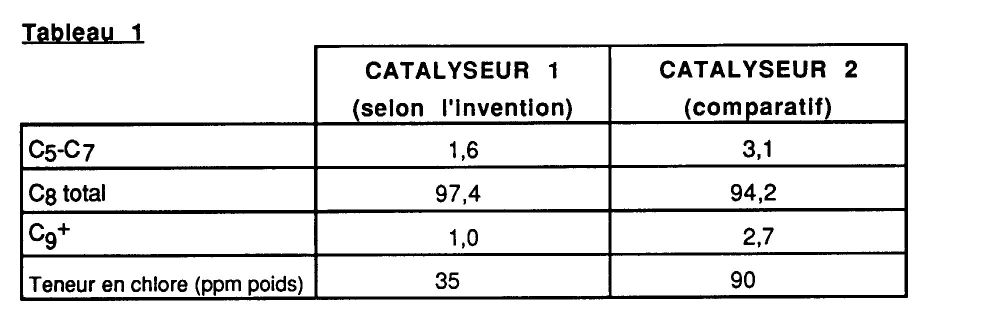

Le tableau 1 met en évidence l'effet de la présence du cuivre(I) dans le catalyseur 1 : le catalyseur 1 conduit, pour des conditions de mise en oeuvre identiques, à une sélectivité en composés C8 plus grande et à une sélectivité en composé lourds C9+, composés indésirables, plus faible. Par ailleurs, le catalyseur 1 conduit à une perte en chlore plus faible que le catalyseur 2.

### Exemple n°3

### Préparation du catalyseur 3 selon l'invention

On active 15 g de silice de volume poreux total égal à 2,2 cm³/g, de surface spécifique égale à 420 m²/g et de diamètre moyen des particules égal à 60 µm, par séchage à 150°C pendant 12 heures. La silice ainsi activée est conservée sous azote. On procède alors à une imprégnation à sec de 10 g de ladite silice par 27,4 g de la phase active préparée dans l'exemple n°1.

Le solide ainsi obtenu, appelé catalyseur 3, possède une teneur pondérale en phase active égale à 73,2 % poids. Il est conservé à l'abri de l'humidité sous argon.

On introduit 15 g du catalyseur 3 préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.

150 cm³ d'isobutane sont alors ajoutés dans le réacteur contenant le catalyseur sous agitation (hélice), ledit réacteur étant immergé dans un bain froid à -5°C. On laisse sous agitation le système (catalyseur 3 + isobutane) pendant une durée de 30 minutes afin d'homogénéiser la température.

On ajoute régulièrement 6,0 g de butène-2 par heure pendant une durée totale de 6 heures, la température du réacteur étant maintenue à -5 °C pendant toute la durée de l'injection.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 2. La conversion de l'oléfine est de 100 %.

### Exemple 4

### Préparation du catalyseur 4 comparatif

On prépare 15 g de silice déshydratée, identique à celle employée dans l'exemple 3, d'une façon identique à la préparation du catalyseur conforme à l'invention de l'exemple 3 ci-dessus. On procède alors à une imprégnation à sec de 10 g de ladite silice par 27,4 g de la phase active préparée dans l'exemple n°2

Le solide ainsi obtenu, appelé catalyseur 4, et possède une teneur pondérale en phase acide égale à 73,2 % poids ; il est conservé à l'abri de l'humidité sous argon.

### Alkylation de l'isobutane par le butènen-2 avec le catalyseur 4

On introduit 15 g du catalyseur 4 préparé selon la méthode décrite ci-dessus dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20°C.

150 cm³ d'isobutane sont alors ajoutés dans le réacteur contenant le catalyseur sous agitation (hélice), ledit réacteur étant immergé dans un bain froid à -5°C. On laisse sous agitation le système (catalyseur 4 + isobutane) pendant une durée de 30 minutes afin d'homogénéiser la température.

On ajoute régulièrement 6,0 g de butène-2 par heure pendant une durée totale de 6 heures, la température du réacteur étant maintenue à -5 °C pendant toute la durée de l'injection.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 2. La conversion de l'oléfine est de 100 %.

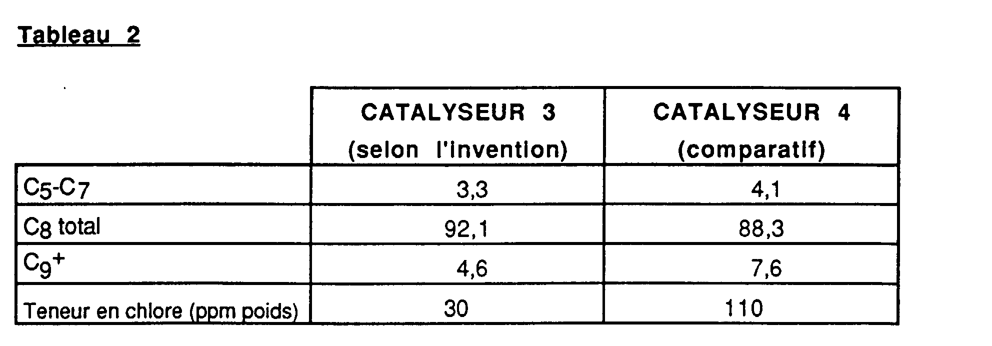

Le tableau 2 met en évidence l'effet de la présence du cuivre(l) dans le catalyseur 3 qui conduit, pour des conditions de mise en oeuvre identiques à une sélectivité en composés C8 plus grande et à une sélectivité en composé lourds C9+, composés indésirables, plus faible. Par ailleurs, le catalyseur 3 conduit à une perte en chlore plus faible que le catalyseur 4.

### Exemple n°5

On introduit 15,3 g du catalyseur 1 préparé selon la méthode décrite dans l'exemple 1 dans un réacteur en acier d'un volume de 500 ml préalablement purgé sous débit d'argon. Le réacteur contenant le catalyseur est alors fermé puis refroidi à la température de - 5°C.

Le réacteur contenant le catalyseur 1 est ensuite rempli d'isobutane séché par passage sur tamis moléculaire. Ledit réacteur est refroidi à - 5°C par circulation d'un liquide froid dans la double enveloppe dont il est équipé. Le catalyseur est alors mis en suspension dans l'isobutane par mise en route du système d'agitation mécanique. Le mobile d'agitation est une turbine et sa vitesse de rotation est de 700 tours par minute. L'agitation est maintenue pendant une durée de 30 minutes afin d'homogénéiser la température.

Puis, toujours sous agitation, on injecte dans le réacteur un mélange constitué de 4,9 % poids de butène-2 et de 95,1 % poids d'isobutane, pendant une durée totale de 300 heures à un débit de 63 g par heure, la température du réacteur étant maintenue à -5 °C pendant toute la durée de l'injection. Le flux sortant du réacteur, qui est composé d'un mélange d'alkylat et d'isobutane, est analysé directement sans procéder à aucune opération d'évaporation. Cette analyse est réalisée après 150 heures et 300 heures de fonctionnement. Les résultats obtenus ainsi que les teneurs en chlore mesurées, après évaporation de l'isobutane, sont reportées dans le tableau 3.

### Exemple n°6

On introduit 15,3 g du catalyseur 2 préparé selon la méthode décrite dans l'exemple 2 dans un réacteur en acier d'un volume de 500 ml préalablement purgé sous débit d'argon. Le réacteur contenant le catalyseur est alors fermé puis il est refroidi à la température de - 5°C.

Puis on réalise le test catalytique dans les mêmes conditions opératoires que celles utilisées dans l'exemple 5. Les analyses sont effectuées aux mêmes durées d'injection de charge. Les résultats obtenus ainsi que les teneurs en chlore mesurées sont reportées dans le tableau 3.

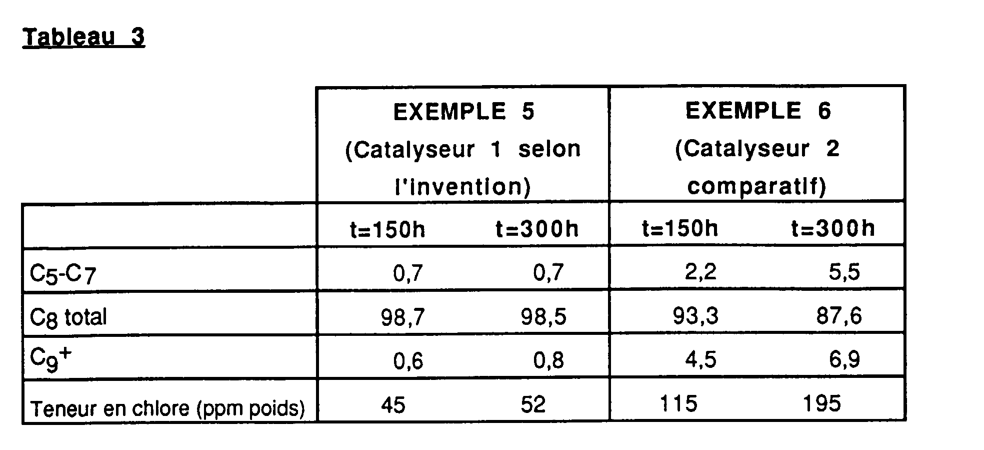

### EXEMPLE N° 7

### Préparation du catalyseur 5 selon l'invention

On active 40 g de silice de surface spécifique égale à 265 m2/g et de volume poreux total égal à 0,25 cm3/g et de diamètre moyen des particules égal à 15 µm, par calcination sous air sec pendant 4 heures à 500°C. La silice ainsi activée est conservée sous argon. On procède alors à une imprégnation à sec et à l'abri de l'humidité, de 30 g de ladite silice par 9,5 g de phase active préparée dans l'exemple 1.

Le solide ainsi préparé, appelé catalyseur 5, possède une teneur pondérale en phase active égale à 24 % poids.

### Alkylation de l'isobutane par le butène-2 avec le catalyseur 5

Le catalyseur 5 est utilisé pour alkyler l'isobutane par le butène-2 de façon à produire des paraffines ramifiées à hauts indices d'octane. On introduit 30 g du catalyseur 5, préparé ci-dessus, dans un réacteur en verre du type Fischer & Porter d'un volume de 360 ml préalablement purgé sous débit d'argon. Le réacteur contenant le catalyseur est alors fermé, mis sous vide primaire, puis il est refroidi à la température de -20 °C.

100 cm³ d'isobutane sont alors ajoutés dans le réacteur contenant le catalyseur sous agitation, ledit réacteur étant immergé dans un bain froid à - 5 °C. On laisse sous agitation le système catalyseur + isobutane pendant une durée de 30 minutes afin d'homogénéiser la température.

On ajoute en continu un mélange d'isobutane et de butène-2, contenant 25 % poids de butène-2, pendant une durée totale de 6 heures, la température du réacteur étant maintenu à -5 °C pendant toute la durée de l'injection. Le débit volumique du mélange (isobutane + butène-2) est égal à 20 ml/h.

Après réaction, la phase hydrocarbure est soutirée du réacteur, puis l'isobutane est évaporé lentement et on recueille l'alkylat qui est analysé par chromatographie en phase vapeur. Sa composition pondérale est donnée dans le tableau 4. La conversion de l'oléfine est de 100 %.

### Exemple n°8

### Préparation du catalyseur 6 comparatif

On active 40 g de silice de surface spécifique égale à 265 m2/g et de volume poreux total égal à 0,25 ml/g et de diamètre moyen des particules égal à 15 µm, par calcination sous air sec pendant 4 heures à 500°C. La silice ainsi activée est conservée sous argon. On procède alors à une imprégnation à sec et à l'abri de l'humidité, de 30 g de ladite silice par 9,5 g de phase active préparée selon l'exemple 2.

Le solide ainsi préparé, appelé catalyseur 6, possède une teneur pondérale en phase active égale à 24 % poids.

Le catalyseur 6 ainsi préparé est testé dans les mêmes conditions opératoires que celles décrites dans l'exemple 1. Les résultats catalytiques obtenus sont donnés dans le tableau 4. La conversion de l'oléfine est de 100%.

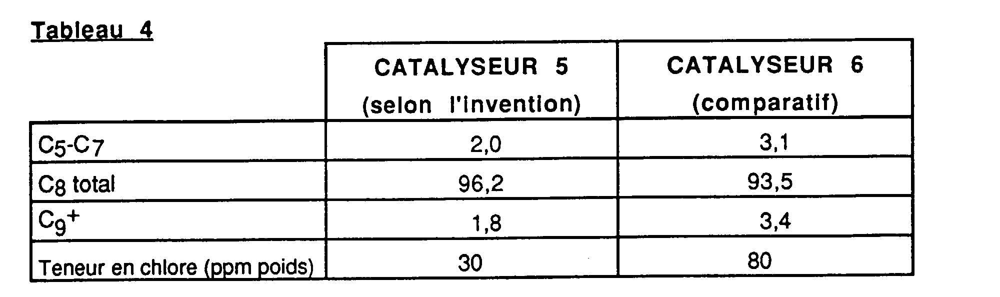

Le tableau 4 met en évidence l'effet de la présence du cuivre(l) dans le catalyseur 5 qui conduit, pour des conditions de mise en oeuvre identiques, à une sélectivité en composés C8 plus grande et à une sélectivité en composé lourds C9+, composés indésirables, plus faible. Par ailleurs, le catalyseur 5 conduit à une perte en chlore plus faible que le catalyseur 6.

## Revendications

1. Catalyseur comprenant un support poreux organique ou minéral et au moins une phase active comprenant au moins un halogénure d'aluminium, au moins un composé choisi parmi les halogénures d'ammonium quaternaire et les halohydrates d'amine, et au moins un composé cuivreux, ledit support ayant été imprégné par ladite phase active, ledit catalyseur étant tel qu'il est constitué essentiellement de particules de diamètre moyen compris entre 0,1 et 150 µm, et en ce que ledit support, avant son imprégnation par ladite phase active, possède un volume poreux total compris entre 0,1 et 6 cm³/g.

2. Catalyseur selon la revendication 1 tel que le composé cuivreux est choisi dans le groupe formé par les halogénures cuivreux, l'acétate cuivreux, le sulfate cuivreux, le nitrate cuivreux et le perchlorate cuivreux.

3. Catalyseur selon l'une des revendications 1 ou 2 tel que le composé cuivreux est un halogénure cuivreux.

4. Catalyseur selon l'une des revendications 1 à 3 tel que le composé cuivreux est choisi dans le groupe constitué par le chlorure de cuivre(l) et le bromure de cuivre(l).

5. Catalyseur selon l'une des revendications 1 à 4 tel que l'halogénure d'aluminium est choisi dans le groupe constitué par le chlorure d'aluminium et le bromure d'aluminium.

6. Catalyseur selon l'une des revendications 1 à 5 tel que l'halogénure d'ammonium quaternaire est choisi dans le groupe formé par les composés de formule générale:
R¹R²R³R⁴N⁺ X⁻ (I)
R¹R²N⁺=CR³R⁴⁺ X⁻ (II) 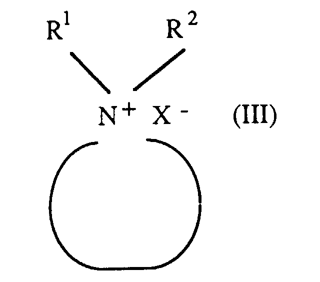 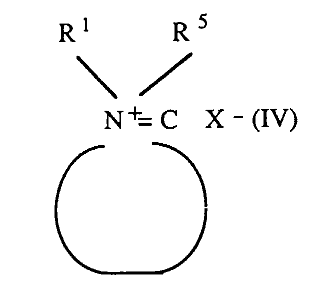
dans lesquelles R¹,R²,R³,R⁴,R⁵ identiques ou différents représentent des restes hydrocarbyles ayant 1 à 12 atomes de carbone, R⁵ pouvant être également l'hydrogène, et dans lesquelles les cycles sont constitués de 4 à 10 atomes, X représentant l'ion halogénure.

7. Catalyseur selon l'une des revendications 1 à 5 tel que l'halogénure d'ammonium quaternaire a pour formule générale:
R¹R²⁺N=CR³-R⁶-R³C=N⁺R¹R² (X⁻)₂
dans laquelle R¹,R²,R³ identiques ou différents représentent des restes hydrocarbyles ayant 1 à 12 atomes de carbone, et R⁶ représente un reste alkylène ou phénylène, X représentant l'ion halogénure.

8. Catalyseur selon l'une des revendications 1 à 6 tel que l'halogénure d'ammonium quaternaire est choisi dans le groupe constitué par le chlorure de N-butylpyridinium, le bromure d'éthylpyridinium, le chlorure de butyl-1 méthyl-3 imidazolium, le chlorure de diéthylpyrazolium, le chlorure d'éthyl-1 méthyl-3 imidazolium.

9. Catalyseur selon l'une des revendications 1 à 6 dans laquelle l'halohydrate d'amine est choisi dans le groupe constitué par les halohydrates comportant une mole d'acide halohydrique par mole d'amine et les halohydrates comportant 2 moles d'acide halohydrique par mole d'amine.

10. Catalyseur selon la revendication 9 tel que l'amine est choisie dans le groupe des composés de formules générales
NR'¹R'²R'³ (I)
R'¹N=CR'²R'³ (II) 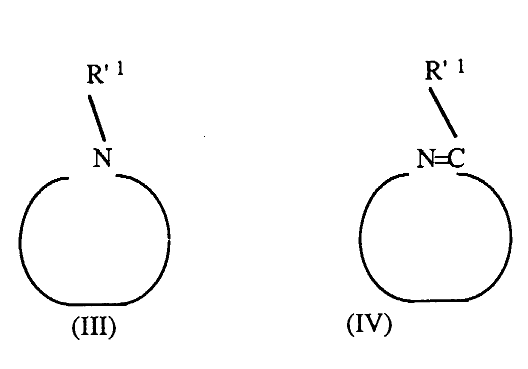
dans lesquelles R'¹,R'²,R'³, identiques ou différents représentent des restes hydrocarbyles ayant 1 à 12 atomes de carbone, et dans lesquelles les cycles sont constitués de 4 à 10 atomes, l'un des substituants R'¹, R'² ou R'³ pouvant être l'hydrogène.

11. Catalyseur selon l'une des revendications 9 ou 10 tel que l'acide halohydrique est choisi dans le groupe formé par l'acide chlorhydrique et l'acide bromhydrique.

12. Catalyseur selon l'une des revendications 1 à 11 tel que l'halohydrate d'amine est choisie dans le groupe formé par les chlorhydrates et es bromhydrates de la pyridine, des picolines-2, -3 et- 4, du N-méthylimidazole, du N-butylimidazole, des lutidines, de l'éthyl-2 pyridine, de l'isopropyl-3 pyridine, des chloro-2 ou -4 pyridine, de la N,N-diméthylamino-4 pyridine, de la pipéridine, de la N-méthylimidazoline.

13. Catalyseur selon l'une des revendications 1 à 12 tel que le rapport molaire entre l'halogénure d'aluminium et l'halogénure d'ammonium quaternaire et/ou l'halohydrate d'amine est compris entre 1,1:1 et 3,5:1.

14. Catalyseur selon l'une des revendications 1 à 13 tel que le rapport molaire entre l'halogénure d'aluminium et l'halogénure cuivreux est compris entre1 :0,1 et 1:1.

15. Catalyseur selon l'une des revendications 1 à 14 tel que ledit support est la silice.

16. Utilisation du catalyseur selon l'une des revendications 1 à 15 en alkylation catalytique d'au moins une isoparaffine choisie dans le groupe formé par l'isobutane et l'isopentane par au moins une oléfine comportant entre 2 et 6 atomes de carbone dans laquelle la température de réaction est comprise entre - 20 et + 25°C et la pression de la zone réactionnelle est suffisante pour maintenir les hydrocarbures à l'état liquide dans ladite zone.

17. Utilisation selon la revendication 16 dans laquelle le catalyseur est mis en oeuvre dans une zone réactionnelle à mélange presque parfait.

18. Utilisation selon la revendication 16 dans laquelle le catalyseur est mis en oeuvre en lit mobile à co-courant.
